# EUROPEAN PATENT APPLICATION

(11) **EP 4 400 150 A1**
(43) Date of publication of application: **17.07.2024**
(21) Application number: 23220709.2
(22) Date of filing: 29.12.2023
(51) Int. Cl.: A61M 25/02

(54) **MANAGEMENT OF CATHETERS AND ACCESSORIES DURING INTRAVASCULAR CATHETER MAINTENANCE**

(30) Priority: 03.01.2023 US 202318092592
(71) Applicant: Becton, Dickinson and Company, Franklin Lakes, NJ 07417 (US)
(72) Inventor: PRASAD, Shishir, Ramsey, NJ 07446 (US); AGDEPPA, Eric Dustin, Cary, NC 27518 (US); TRIPATHI, Sandeep, New York, NY 10025 (US); YOUNG, Duncan, Melbourn, SG8 6FE (GB); HAMLYN, William, Royston, SG8 5AL (GB); LYONS, Simon, Cottenham, CB24 8AU (GB); KILBY, Douglas, London, N19JL (GB); SADLER, Matthew, Bury St. Edmunds, IP28 7PX (GB); LIPSCOMBE, Daniel, London, TW1 2DE (GB)
(74) Representative: dompatent von Kreisler Selting Werner - Partnerschaft von Patent- und Rechtsanwälten mbB

(57) **Abstract**

An intravenous catheter securement device includes a holder for placement on a bodily appendage near an intravenous insertion site on the bodily appendage, a strap for securing the holder to the bodily appendage, and at least one securement feature formed on the holder and configured to secure at least a portion of an intravenous catheter. The holder is shaped to surround at least a portion of the bodily appendage. The at least one securement feature is a slot formed on the holder. The holder includes a tray having an interior cavity and a tearable lid covering the interior cavity.

## Description

### CROSS-REFERENCE TO RELATED APPLICATION

The present application claims priority to United States Utility Application No. 18/092,592 entitled "Management of Catheters and Accessories During Intravascular Catheter Maintenance" filed January 3, 2023, the entire disclosure of which is hereby incorporated by reference in its entirety.

### BACKGROUND

### Technical Field

**The** present disclosure relates to a device for managing catheters and accessories. More specifically, the present disclosure relates to a device for managing catheters and accessories during and post intravascular catheter access.

### Technical Considerations

Intravenous (IV) therapy refers to a medical technique that is used to deliver fluids, medications, and/or nutrition directly into a body of a patient via an IV catheter inserted into a vein at an IV insertion site. IV therapy may be used for rehydration, to provide nutrition to a person who cannot consume food or water by mouth, and/or to administer medications or other medical therapy, such as blood products or electrolytes.

After an IV catheter is inserted at an IV insertion site, the IV catheter must be secured from being accidentally pulled. While a variety of solutions exist for securing IV catheters and maintaining the IV insertion site, clinicians are often left with limited solutions to manage the loose portions of the IV catheter lumen that extend outside of the patient's body. These loose extensions can create opportunities for the lumen to be pulled, potentially compromising the catheter position or insertion site integrity. Tape and textile dressings are sometimes used, but can be cumbersome when the catheter needs to be accessed. In addition, while the catheter is being accessed, an un-capped line is at risk for touch contamination. The presence of multiple IV catheters and/or various accessories used with IV catheters often complicates patient care because the unused catheters and accessories must be moved out of the way for accessing the desired IV catheter.

Accordingly, there is an increasing need for management of IV catheters and accessories. Specifically, there is an increasing need for management of IV catheters and accessories during and post intravascular catheter access.

### SUMMARY

Accordingly, provided are improved systems, devices, products, apparatus, and/or methods for management of IV catheters and accessories during and post intravascular catheter access.

In some non-limiting embodiments or aspects, provided is an IV catheter securement device that may include a holder for placement on a bodily appendage near an IV insertion site on the bodily appendage; a strap for securing the holder to the bodily appendage; and at least one securement feature formed on the holder and configured to secure at least a portion of an IV catheter. The holder may be shaped to surround at least a portion of the bodily appendage.

In some non-limiting embodiments or aspects, the at least one securement feature may be a slot formed on the holder. The holder may include a tray having an interior cavity and a tearable lid covering the interior cavity. The tray may have a foam or similarly soft and flexible insert in at least a portion of the interior cavity. The tray may have at least one catheter accessory holder slot.

In some non-limiting embodiments or aspects, at least one tearable or easily removable strap may be removably secured to the holder. The at least one tearable or easily removable strap may have at least one opening configured to receive at least a portion of bodily appendage. The holder is made of foam or similar soft and flexible material made of plastic or elastomer.

In some non-limiting embodiments or aspects, at least one monitoring device may be connected to the holder and configured to detect at least one functional parameter. The at least one monitoring device may include at least one sensor configured to detect the at least one functional parameter that is a physiological parameter of the patient. The at least one sensor may be at least one of a moisture sensor; a temperature sensor; a blood pressure sensor; an accelerometer; and any combination thereof.

In some non-limiting embodiments or aspects, a communication device may be in operative communication with the at least one monitoring device, the communication device configured for sending data from the at least one monitoring device to a patient or a remote site.

In some non-limiting embodiments or aspects, at least one remedy device may be connected to the holder and configured to administer a remedy. The at least one remedy device may be a massager configured to stimulate an area around the insertion site.

In some non-limiting embodiments or aspects, the intravenous catheter securement device may include an IV catheter having a first end configured for connection to the IV insertion site and a second end configured for connection to at least one of the plurality of securement features formed on the holder.

In some non-limiting embodiments or aspects, an IV catheter securement device may include a holder for placement on a bodily appendage near an IV insertion site on the bodily appendage, the holder comprising a tray having an interior cavity and a tearable lid covering the interior cavity; a strap for securing the holder to the bodily appendage; and at least one securement feature formed on the tray and configured to secure at least a portion of an IV catheter or a syringe.

In some non-limiting embodiments or aspects, at least one securement feature may be at least one slot. At least one tearable or easily removable strap may be removably secured to the holder. The at least one tearable or easily removable strap may have at least one opening configured to receive at least a portion of bodily appendage. The tray may have a foam insert in at least a portion of the interior cavity.

Additional embodiments or aspects of the improved systems, devices, products, apparatus, and/or methods for management of IV catheters and accessories during and post intravascular catheter access are detailed in one or more of the following clauses:
Clause 1: An intravenous catheter securement device comprising: a holder for placement on a bodily appendage near an intravenous insertion site on the bodily appendage; a strap for securing the holder to the bodily appendage; and at least one securement feature formed on the holder and configured to secure at least a portion of an intravenous catheter, wherein the holder is shaped to surround at least a portion of the bodily appendage.
Clause 2: The intravenous catheter securement device of clause 1, wherein the at least one securement feature is an expandable slot formed on the holder.
Clause 3: The intravenous catheter securement device of clause 1 or 2, wherein the holder comprises a tray having an interior cavity and a tearable lid covering the interior cavity.
Clause 4: The intravenous catheter securement device of clause 3, wherein the tray has a foam insert in at least a portion of the interior cavity.
Clause 5: The intravenous catheter securement device of clause 3 or 4, wherein the tray comprises at least one catheter accessory holder slot.
Clause 6: The intravenous catheter securement device of any of clauses 1 to 5, further comprising at least one tearable or easily removable strap removably secured to the holder.
Clause 7: The intravenous catheter securement device of any of clauses 1 to 6, wherein the at least one tearable or easily removable strap has at least one opening configured to receive at least a portion of bodily appendage.
Clause 8: The intravenous catheter securement device of any of clauses 1 to 7, wherein the holder is made of foam.
Clause 9: The intravenous catheter securement device of any of clauses 1 to 8, further comprising at least one monitoring device connected to the holder and configured to detect at least one functional parameter.
Clause 10: The intravenous catheter securement device of any of clauses 1 to 9, wherein at least one monitoring device comprises at least one sensor configured to detect the at least one functional parameter that is a physiological parameter of the patient.
Clause 11: The intravenous catheter securement device of clause 10, wherein the at least one sensor is at least one of a moisture sensor; a temperature sensor; a blood pressure sensor; an accelerometer; and any combination thereof.
Clause 12: The intravenous catheter securement device of any of clauses 1 to 11, further comprising a communication device in operative communication with the at least one monitoring device, the communication device configured for sending data from the at least one monitoring device to a patient or a remote site.
Clause 13: The intravenous catheter securement device of any of clauses 1 to 12, further comprising at least one remedy device connected to the holder and configured to administer a remedy.
Clause 14: The intravenous catheter securement device of clause 13, wherein the at least one remedy device is a massager configured to stimulate an area around the insertion site
Clause 15: The intravenous catheter securement device of any of clauses 1 to 14, further comprising the intravenous catheter having a first end configured for connection to the intravenous insertion site and a second end configured for connection to at least one of the plurality of securement features formed on the holder.
Clause 16: An intravenous catheter securement device comprising: a holder for placement on a bodily appendage near an intravenous insertion site on the bodily appendage, the holder comprising a tray having an interior cavity and a tearable lid covering the interior cavity.; a strap for securing the holder to the bodily appendage; and at least one securement feature formed on the tray and configured to secure at least a portion of an intravenous catheter or a syringe.
Clause 17: The intravenous catheter securement device of clause 16, wherein at least one securement feature is at least one slot.
Clause 18: The intravenous catheter securement device of clause 16 or 17, further comprising at least one tearable or easily removable strap removably secured to the holder.
Clause 19: The intravenous catheter securement device of clause 18, wherein the at least one tearable or easily removable strap has at least one opening configured to receive at least a portion of bodily appendage.
Clause 20: The intravenous catheter securement device of any of clauses 16-19, wherein the tray has a foam insert in at least a portion of the interior cavity.

### BRIEF DESCRIPTION OF THE DRAWINGS

Additional advantages and details are explained in greater detail below with reference to the exemplary embodiments that are illustrated in the accompanying schematic figures, in which:
FIG. 1 is an illustration of an IV catheter securement device worn by a patient in accordance with one embodiment or aspect of the present disclosure;
FIG. 2 is a perspective view of the IV catheter securement device shown in FIG. 1;
FIG. 3 is a perspective view of an IV catheter securement device in accordance with another embodiment or aspect of the present disclosure;
FIG. 4 is a perspective view of an IV catheter securement device in accordance with another embodiment or aspect of the present disclosure;
FIG. 5 is a perspective view of an IV catheter securement device in accordance with another embodiment or aspect of the present disclosure;
FIG. 6 is a perspective view of an IV catheter securement device in accordance with another embodiment or aspect of the present disclosure;
FIG. 7 is a perspective view of an IV catheter securement device in accordance with another embodiment or aspect of the present disclosure;
FIG. 8 is a perspective view of an IV catheter securement device in accordance with another embodiment or aspect of the present disclosure;
FIG. 9 is a perspective view of an IV catheter securement device in accordance with another embodiment or aspect of the present disclosure;
FIG. 10 is a perspective view of an IV catheter securement device in accordance with another embodiment or aspect of the present disclosure;
FIG. 11 is a top perspective view of an IV management holder for use with an IV catheter securement device shown in accordance with an embodiment or aspect of the present disclosure;
FIG. 12 is a bottom perspective view of the IV management holder shown in FIG. 11;
FIG. 13 is a top perspective view of an IV management tray for use with an IV catheter securement device shown in accordance with another embodiment or aspect of the present disclosure;
FIG. 14 is a detailed view of a portion of the IV management holder shown in FIG. 13;
FIG. 15 is a top perspective view of an IV management holder for use with an IV catheter securement device shown in accordance with another embodiment or aspect of the present disclosure; and
FIGS. 16-19 are illustrations of various embodiments of an IV management holder in use on a patient's hand.

### DETAILED DESCRIPTION

It is to be understood that the present disclosure may assume various alternative variations and step sequences, except where expressly specified to the contrary. It is also to be understood that the specific devices and processes illustrated in the attached drawings, and described in the following specification, are simply exemplary and non-limiting embodiments or aspects. Hence, specific dimensions and other physical characteristics related to the embodiments or aspects disclosed herein are not to be considered as limiting.

For purposes of the description hereinafter, the terms "end," "upper," "lower," "right," "left," "vertical," "horizontal," "top," "bottom," "lateral," "longitudinal," and derivatives thereof shall relate to embodiments or aspects as they are oriented in the drawing figures. However, it is to be understood that embodiments or aspects may assume various alternative variations and step sequences, except where expressly specified to the contrary. It is also to be understood that the specific devices and processes illustrated in the attached drawings, and described in the following specification, are simply non-limiting exemplary embodiments or aspects. Hence, specific dimensions and other physical characteristics related to the embodiments or aspects of the embodiments or aspects disclosed herein are not to be considered as limiting unless otherwise indicated.

No aspect, component, element, structure, act, step, function, instruction, and/or the like used herein should be construed as critical or essential unless explicitly described as such. Also, as used herein, the articles "a" and "an" are intended to include one or more items, and may be used interchangeably with "one or more" and "at least one." As used in the specification and the claims, the singular form of "a," "an," and "the" include plural referents, such as unless the context clearly dictates otherwise. Additionally, Furthermore, as used herein, the terms "set" and "group" are intended to include one or more items (e.g., related items, unrelated items, a combination of related and unrelated items, etc.) and may be used interchangeably with "one or more" or "at least one." Where only one item is intended, the term "one" or similar language is used. Also, as used herein, the terms "has," "have," "having," or the like are intended to be open-ended terms. Further, the phrase "based on" is intended to mean "based at least partially on" unless explicitly stated otherwise. Further, the phrase "based on" is intended to mean "based at least partially on" unless explicitly stated otherwise. Further, the phrase "based on" may mean "in response to" and be indicative of a condition for automatically triggering a specified operation of an electronic device (e.g., a controller, a processor, a computing device, etc.) as appropriately referred to herein.

All numbers used in the specification and claims are to be understood as being modified in all instances by the term "about". The terms "approximately", "about", and "substantially" mean a range of plus or minus ten percent of the stated value.

As used herein, the term "at least one of" is synonymous with "one or more of". For example, the phrase "at least one of A, B, and C" means any one of A, B, and C, or any combination of any two or more of A, B, and C. For example, "at least one of A, B, and C" includes one or more of A alone; or one or more of B alone; or one or more of C alone; or one or more of A and one or more of B; or one or more of A and one or more of C; or one or more of B and one or more of C; or one or more of all of A, B, and C. Similarly, as used herein, the term "at least two of" is synonymous with "two or more of". For example, the phrase "at least two of D, E, and F" means any combination of any two or more of D, E, and F. For example, "at least two of D, E, and F" includes one or more of D and one or more of E; or one or more of D and one or more of F; or one or more of E and one or more of F; or one or more of all of D, E, and F.

Some non-limiting embodiments or aspects may be described herein in connection with thresholds. As used herein, satisfying a threshold may refer to a value being greater than the threshold, more than the threshold, higher than the threshold, greater than or equal to the threshold, less than the threshold, fewer than the threshold, lower than the threshold, less than or equal to the threshold, equal to the threshold, etc.

Provided are improved devices, systems, methods, and products for an IV catheter securement device configured for safeguarding the insertion site and connectors of the IV catheter, permitting maintenance of the IV catheter, and monitoring of at least one patient characteristic.

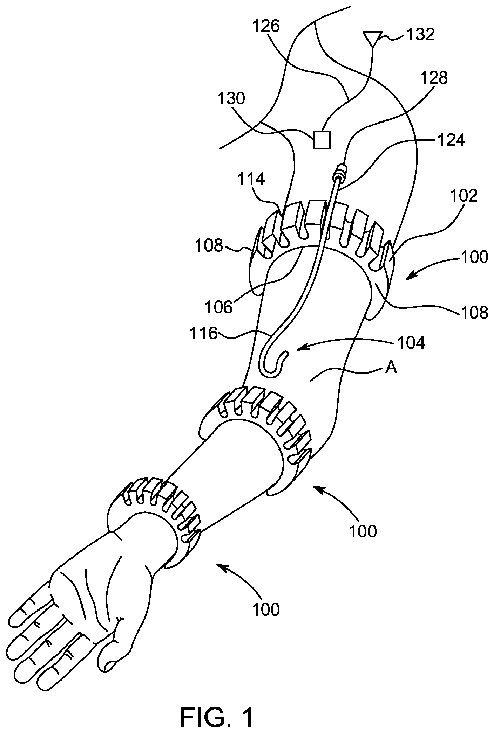

Referring now to FIGS. 1-2, an intravenous ("IV") catheter securement device 100 is shown in accordance with various embodiments or aspects. The IV catheter securement device 100 has a holder 102 for placement on a bodily appendage A. The holder 102 is shaped to surround at least a portion of the bodily appendage A. In various embodiments or aspects, the holder 102 can be configured to be worn on the bodily appendage A near an IV catheter insertion site 104. For example, and with reference to FIG. 1, the holder 102 can be configured as a cuff surrounding at least a portion of the patient's arm. In other embodiments or aspects, the holder 102 may be configured to surround at least a portion of the patient's leg, torso, neck, or any other bodily appendage A.

The holder 102 can be formed as pre-shaped structure made from a foam material. In some embodiments or aspects, the holder 102 is made from a flexible plastic material. Desirably, the holder 102 is made from a material that is easy to clean.

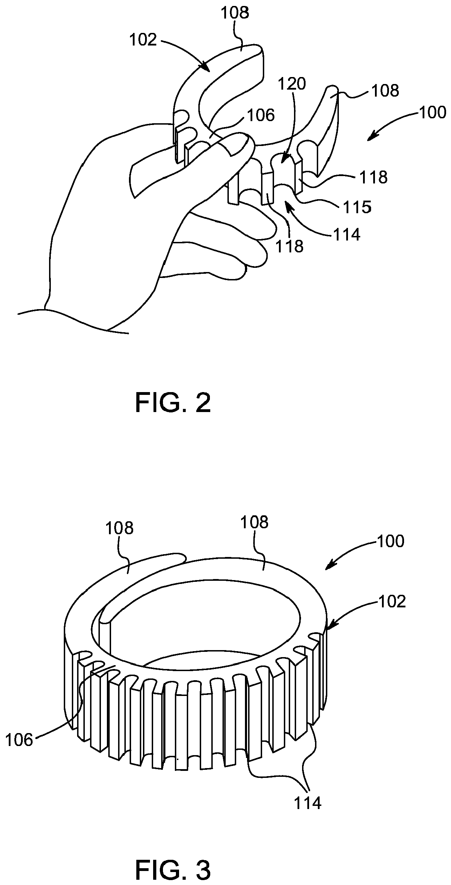

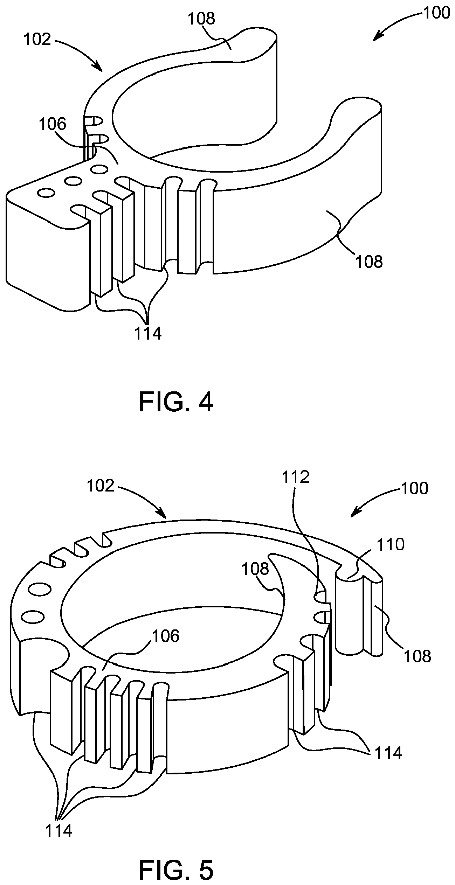

**The** holder 102 may have a body 106 with at least one strap 108 extending from the body 106. In embodiments or aspects where two straps 108 are provided, the straps 108 may be deflectable relative to each other to permit placement and removal of the holder 102 to and from the bodily appendage A. In some embodiments or aspects, the straps 108 may at least partially overlap each other, such as shown in FIG. 3. The body 106 and the straps 108 may be shaped to form an arcuate shape. In other embodiments or aspects, and with reference to FIG. 4, the body 106 of the holder 102 may protrude in a direction away from the straps 108. In further embodiments or aspects, the straps 108 may be configured to connect to each other. For example, and with reference to FIG. 5, a first locking element 110 may be provided on one of the straps 108 and a second locking element 112 may be provided on the other of the straps 108. The first and second locking elements 110, 112 may be configured for releasably connecting to each other in order to removably secure the holder 102 to the bodily appendage A. In some embodiments or aspects, the first and locking elements 110, 112 may be clips, clasps, hook and loop fasteners, adhesive, groove/protrusion arrangements, magnets, or any other mechanical fastening arrangement configured to releasably connect the straps 108 to each other.

With reference to FIGS. 1-5, the holder 102 has at least one securement feature 114 formed thereon. The at least one securement feature 114 may be configured to secure at least a portion of an IV catheter 116 to the holder 102. In some embodiments or aspects, a plurality of securement features 114 may be spaced apart along the holder 102, with each of the securement features 114 being configured to secure a separate IV catheter 116 or a related medical accessory for use with the catheter 116. In this manner, the securement features 114 help in maintenance of the IV catheter 116 and the related accessories, such as one or more syringes or a disinfecting unit. The plurality of securement features 114 may be spaced apart from each other at even or uneven intervals.

As shown in FIG. 2, the at least one securement feature 114 may be shaped as a slot 115 having a pair of deflectable arms 118 and a receiving cavity 120 defined between the arms 118. The arms 118 may be deflectable relative to each other to permit insertion of the IV catheter 116 (shown in FIG. 1) into the receiving cavity 120. When the IV catheter 116 is inserted into the receiving cavity 120, the arms 118 may spring back to their undeflected shape to help retain the IV catheter 116 in the receiving cavity 120. This manner, the at least one securement feature 114 is configured to retain the catheter line to prevent the line from being accidentally pulled out of the IV insertion point 104 or maintain the IV connector clean post the disinfection step.

With reference to FIG. 1, the IV catheter securement device 100 includes the IV catheter 116 having a first end 122 configured for connection to the IV insertion site 104 and a second end 124 configured for connection to an IV delivery line 126. The IV catheter 116 may have a predetermined length to allow for the IV catheter 116 to be inserted into a lumen of a blood vessel of a user to allow for IV delivery of fluids. In some embodiments or aspects, the second end 124 may be removably connectable to the IV delivery line. A first coupling 128 may be provided at the second end 124 for removably connecting to a corresponding second coupling 130 at the IV delivery line. In some embodiments or aspects, the first and second couplings 128, 130 may be LuerLoc fluid fittings. In further embodiments or aspects, the IV catheter securement device 100 may be configured for use with other long term access devices, such as urinary catheters.

In some non-limiting embodiments, the IV delivery line 126 may allow fluid to flow from a fluid source connected to the IV delivery line 126 into the IV catheter 116 for delivering the fluid from the fluid source to the patient. As shown in FIG. 1, the IV delivery line 126 may include a connector 132 at an end opposite to the second coupling 130. In some non-limiting embodiments or aspects, the connector 132 may include one or more fittings, such as a LuerLoc fluid fitting. In some non-limiting embodiments or aspects, the connector 132 may be configured to connect to an external fluid source for administration of fluid through the IV catheter 116. In further embodiments or aspects, the connector 132 may be configured to connect to a flushing arrangement for flushing at least one of the IV delivery line 126 and the IV catheter 116 or at least one remedy device for delivering a remedy to the patient, as described herein.

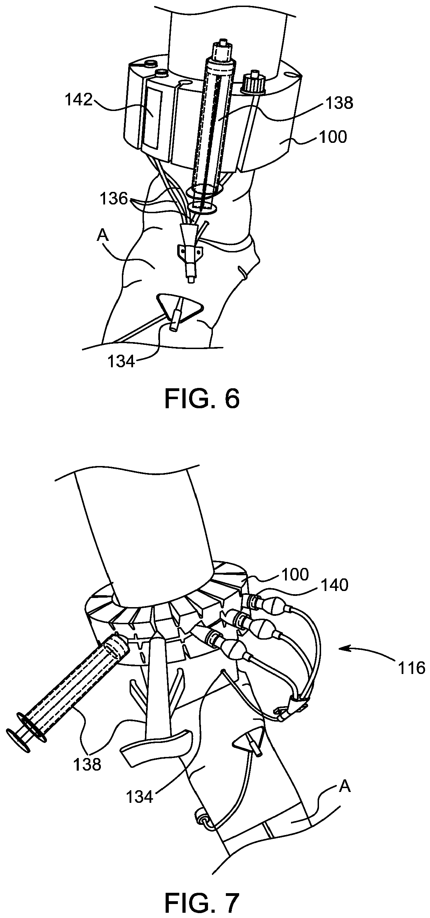

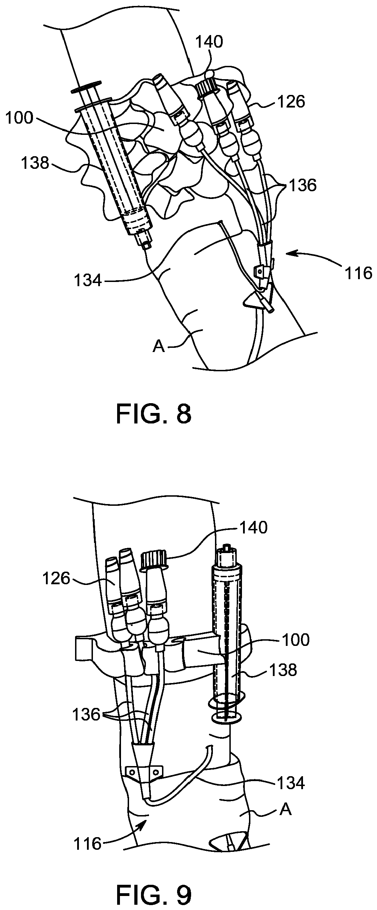

With reference to FIGS. 6-9, various embodiments or aspects of the holder 102 are shown. In each of FIGS. 6-9, the IV catheter 116 has a central line 134 that is inserted into the patient and a plurality of branches 136 that connect to various accessories, such as an IV delivery line 126, a syringe 138, or a connection point 140. In some embodiments or aspects, the holder 102 may have at least one indicia 142. For example, the at least one indicia 142 may contain medication information, nurse notes, patient information, emergency care information, or other information. Multiple indica may be provided next to each braches 136 to provide easily accessible information about each branch to the patient or care providers.

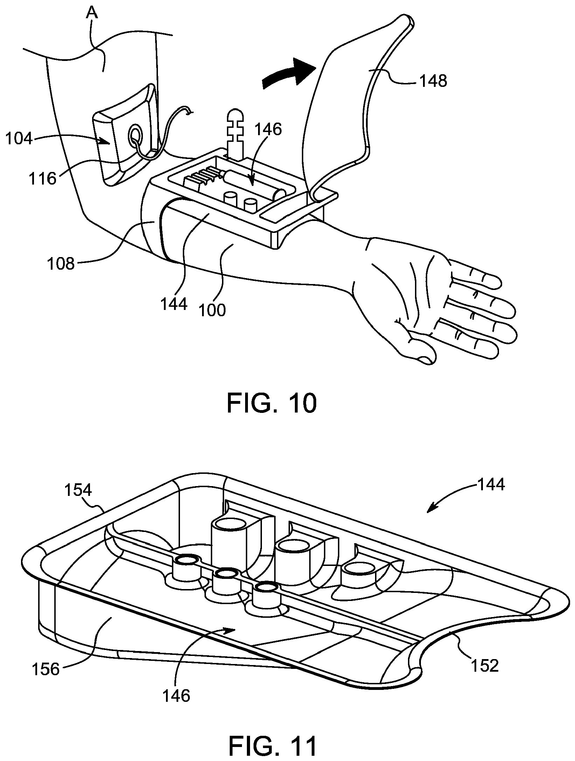

With reference to FIG. 10, the holder 102 includes a tray 144 that is securely mountable to the bodily appendage via the strap 108. The tray 144 has an interior cavity 146 configured for receiving medical equipment, such as at least one syringe, IV catheter, tubing, sterilizing pads, connectors, and/or handling and use instructions. In this manner, the tray 144 can be used for transporting the medical equipment and to organize and hold the medical equipment during use. The medical equipment can be provided as a kit, such as a flush kit or a dressing change kit, with appropriate supplies for the desired procedure. The tray 144 may have a tearable lid 148 covering the interior cavity 146 to retain the medical equipment in the interior cavity 146 prior to use. The interior cavity 146 and the medical equipment may be sterilized prior to or after sealing the interior cavity 146 with the tearable lid 148. In this case, the tearable lid 148 also maintains the sterility of the enclosed medical device prior to the use of the kit.

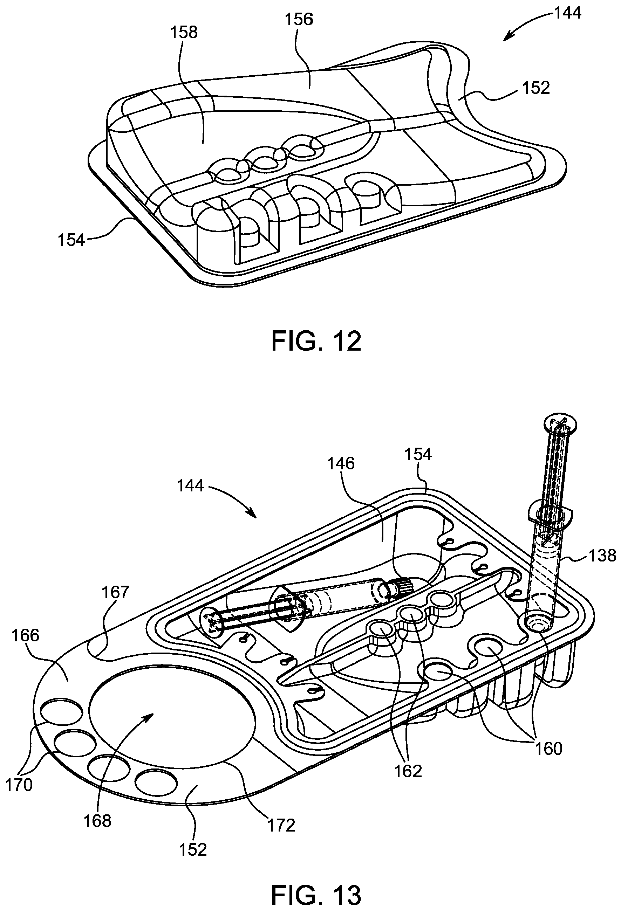

With reference to FIGS. 11-12, the tray 144 may be a vacuum-formed blister pack formed or injection molded from a plastic material. The tray 144 may have a shallow proximal end 152 and a deep distal end 154. With reference to FIG. 12, a bottom surface 156 of the tray 144 may have an anatomically-shaped support surface 158 configured to confirm to the shape of the bodily appendage A (shown in FIG. 1). In some embodiments or aspects, the support surface 158 may have an adhesive or a very soft deformable surface of low hardness (<80 Shore A) to help secure the tray 144 to the bodily appendage A.

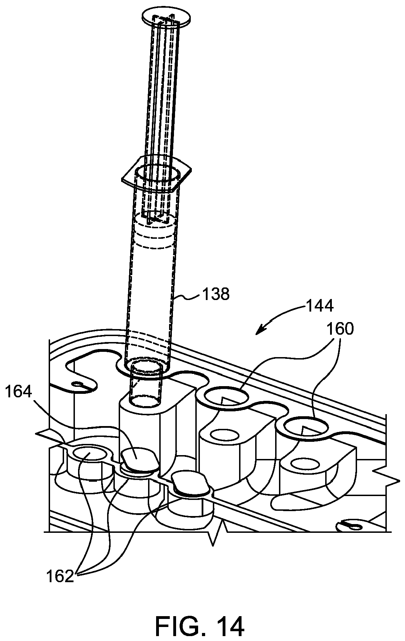

With reference to FIGS. 13-14, the tray 144 may include at least one catheter accessory holder slot 160 configured to hold a catheter accessory, such as a syringe 138, during maintenance of the IV catheter 116 (shown in FIG. 1). The tray 144 further may include one or more chambers 162 pre-filled with a fluid, such as a flushing fluid. The one or more chambers 162 may be sealed with a peelable lid 164. At least one securement feature 114 may be configured to secure at least a portion of an IV catheter 116 (shown in FIG. 1) to the tray 144. In addition, the tray also would have disinfecting devices, connector caps, and gloves.

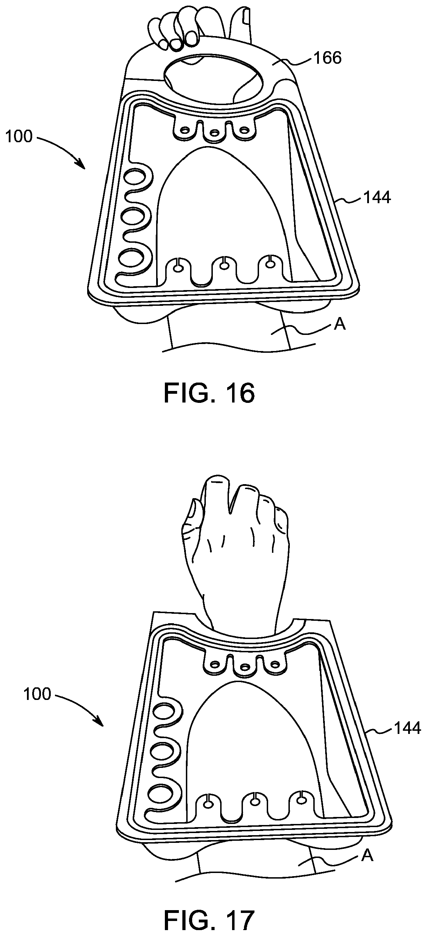

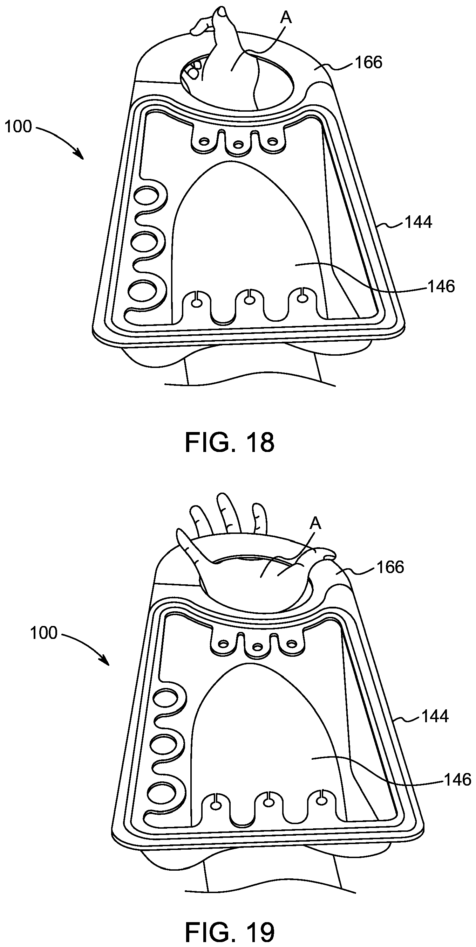

With reference to FIG. 13, the tray 144 may have at least one tearable or easily removable strap 166 removably secured to the holder 102. The at least one tearable or easily removable strap 166 may be removable from the tray 144 by tearing the tearable or easily removable strap 166 at a perforation 167 connecting the tearable or easily removable strap 166 to the tray 144. The at least one tearable or easily removal strap 166 may be foldable relative to the tray 144, such as the perforation 167, for a better anatomical fit on the bodily appendage A (shown in FIGS. 16-19). The at least one tearable or easily removable strap 166 has at least one opening 168 configured to receive at least a portion of bodily appendage A. For example, the at least one opening 168 may be a plurality of finger holes 170 or a hand opening 172. Various illustrations showing use of the tray 144 with the at least one tearable or easily removable strap 166 are shown in FIGS. 16-19.

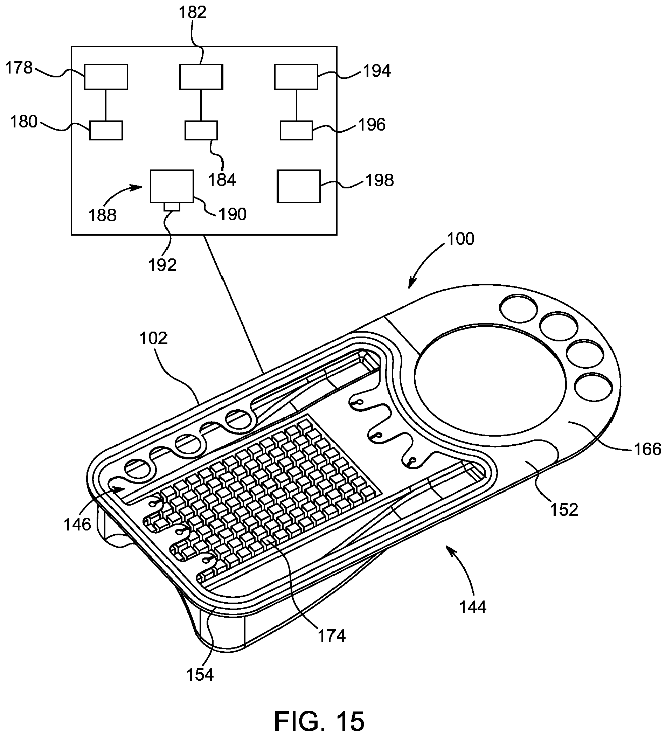

With reference to FIG. 15, the tray 144 may have a foam insert 174 in at least a portion of the interior cavity 146. In some embodiments or aspects, the interior cavity 146 and/or the foam insert 174 may be impregnated with a disinfecting fluid, such as isopropyl alcohol.

With continued reference to FIG. 15, the IV catheter securement device 100 may have at least one monitoring device 178 connected to the holder 102 and configured to detect at least one functional parameter. The at least one monitoring device 178 may be positioned within the interior cavity 146 of the tray 144.

In some embodiments or aspects, the at least one monitoring device 178 may have at least one sensor 180 configured to detect the at least one functional parameter, which may be a physiological parameter of the patient. The at least one sensor 180 may be configured to detect the at least one functional parameter relating to a physiological condition of the patient. In some embodiments or aspects, the at least one sensor 180 may be configured to detect the at least one functional parameter through direct or indirect contact with the patient's skin. In other embodiments or aspects, the at least one sensor 180 may be configured to detect the at least one functional parameter via direct or indirect access to the patient's vasculature.

In some embodiments or aspects, the at least one sensor 180 may be a moisture sensor. The moisture sensor may be configured to detect moisture in an area of the insertion site 104, which may indicate a leak from the insertion site 104. Identifying a leak from the insertion site 104 may be useful in diagnosing an infection, infiltration below the skin, and/or extravasation.

In some embodiments or aspects, the at least one sensor 180 may be a temperature sensor. The temperature sensor may be configured to measure body temperature at the insertion site 104 via direct or indirect contact with the patient's skin. In some embodiments or aspects, the temperature sensor may be configured to measure a temperature differential between the insertion site 104 and a remote site distant from the insertion site 104. A local increase in temperature at the insertion site 104 may be useful in diagnosing an infection, infiltration below the skin, and/or extravasation.

In some embodiments or aspects, the at least one sensor 180 may be a blood pressure sensor. The blood pressure sensor may have IV access via, for example, the IV catheter 116.

In some embodiments or aspects, the at least one sensor 180 may be an accelerometer configured for detecting patient motion. Output of the accelerometer may be useful in determining whether the patient is in a period of extended inactivity, which may lead to muscle soreness.

With continued reference to FIG. 15, the IV catheter securement device 100 may have at least one remedy device 182 connected to holder 102 and configured to administer a remedy. The least one remedy device 182 may be positioned within the interior cavity 146 of the tray 144. In some embodiments or aspects, the at least one remedy device 182 may be a massager 184 configured to stimulate an area around the insertion site 104 to reduce muscle pain or soreness due to long term insertion of the IV catheter 116.

With continued reference to FIG. 15, the IV catheter securement device 100 may have a power source 188 for providing power to the at least one monitoring device 178 and the at least one remedy device 182. In some embodiments or aspects, the power source 188 may be a rechargeable battery 190 having a charging port 192.

With continued reference to FIG. 15, the IV catheter securement device 100 may have a communication device 194 in operative communication with the at least one monitoring device 178. The communication device 194 may be configured for sending data from the at least one monitoring device 178 to the patient or a remote site. In some embodiments or aspects, the IV catheter securement device 100 may have a display 196 for displaying data received by the communication device 194 to the patient. In other embodiments or aspects, data collected by the communication device 194 may be sent wirelessly to a remote terminal, such as using Wi-Fi or Bluetooth data communication protocols.

With continued reference to FIG. 15, the IV catheter securement device 100 may have a controller 198 for controlling operation of the at least one monitoring device 178 and/or the at least one remedy device 182. In some embodiments or aspects, the controller 198 may include a device configured to control (e.g., control the activation and/or deactivation of) the at least one monitoring device 178 and/or the at least one remedy device 182. In some non-limiting embodiments, the controller 198 may include a processor, such as a central processing unit (CPU), a microcontroller, an integrated circuit (IC), and/or the like. In some non-limiting embodiments, the controller 198 may include a switch device, such as an electrical switch.

In some non-limiting embodiments, the at least one monitoring device 178 and/or the at least one remedy device 182 may be controlled based on a sensor. For example, the controller 198 may receive a signal from the sensor and the controller 198 may cause the at least one monitoring device 178 and/or the at least one remedy device 192 to activate or deactivate based on the signal received from the sensor.

In some non-limiting embodiments, the at least one monitoring device 178 and/or the at least one remedy device 182 may be controlled based on a time interval (e.g., a predetermined time interval, a time interval associated with a flushing or infusion process, etc.).

In some non-limiting embodiments, the at least one monitoring device 178 and/or the at least one remedy device 182 may be controlled based on a predetermined sequence. For example, one or more light elements of the at least one monitoring device 178 and/or the at least one remedy device 182 may be activated and/or deactivated based on the predetermined sequence. In some non-limiting embodiments, the at least one monitoring device 178 and/or the at least one remedy device 182 may be controlled based on an input from a user.

Although embodiments or aspects have been described in detail for the purpose of illustration and description, it is to be understood that such detail is solely for that purpose and that embodiments or aspects are not limited to the disclosed embodiments or aspects, but, on the contrary, are intended to cover modifications and equivalent arrangements that are within the spirit and scope of the appended claims. For example, it is to be understood that the present disclosure contemplates that, to the extent possible, one or more features of any embodiment or aspect can be combined with one or more features of any other embodiment or aspect. In fact, many of these features can be combined in ways not specifically recited in the claims and/or disclosed in the specification. Although each dependent claim listed below may directly depend on only one claim, the disclosure of possible implementations includes each dependent claim in combination with every other claim in the claim set.

## Claims

1. An intravenous catheter securement device comprising:
a holder for placement on a bodily appendage near an intravenous insertion site on the bodily appendage;
a strap for securing the holder to the bodily appendage; and
at least one securement feature formed on the holder and configured to secure at least a portion of an intravenous catheter,
wherein the holder is shaped to surround at least a portion of the bodily appendage.

2. The intravenous catheter securement device of claim 1, wherein the at least one securement feature is a slot formed on the holder.

3. The intravenous catheter securement device of claim 1, wherein the holder comprises a tray having an interior cavity and a tearable lid covering the interior cavity.

4. The intravenous catheter securement device of claim 3, wherein the tray has a foam insert in at least a portion of the interior cavity.

5. The intravenous catheter securement device of claim 3, wherein the tray comprises at least one catheter accessory holder slot.

6. The intravenous catheter securement device of claim 1, further comprising at least one tearable or easily removable strap removably secured to the holder.

7. The intravenous catheter securement device of claim 1, wherein the at least one tearable or easily removable strap has at least one opening configured to receive at least a portion of bodily appendage.

8. The intravenous catheter securement device of claim 1, wherein the holder is made of foam.

9. The intravenous catheter securement device of claim 1, further comprising at least one monitoring device connected to the holder and configured to detect at least one functional parameter.

10. The intravenous catheter securement device of claim 1, wherein at least one monitoring device comprises at least one sensor configured to detect the at least one functional parameter that is a physiological parameter of the patient.

11. The intravenous catheter securement device of claim 10, wherein the at least one sensor is at least one of a moisture sensor; a temperature sensor; a blood pressure sensor; an accelerometer; and any combination thereof.

12. The intravenous catheter securement device of claim 1, further comprising a communication device in operative communication with the at least one monitoring device, the communication device configured for sending data from the at least one monitoring device to a patient or a remote site.

13. The intravenous catheter securement device of claim 1, further comprising at least one remedy device connected to the holder and configured to administer a remedy.

14. The intravenous catheter securement device of claim 13, wherein the at least one remedy device is a massager configured to stimulate an area around the insertion site.

15. The intravenous catheter securement device of claim 1, further comprising the intravenous catheter having a first end configured for connection to the intravenous insertion site and a second end configured for connection to at least one of the plurality of securement features formed on the holder.

16. An intravenous catheter securement device comprising:
a holder for placement on a bodily appendage near an intravenous insertion site on the bodily appendage, the holder comprising a tray having an interior cavity and a tearable lid covering the interior cavity;
a strap for securing the holder to the bodily appendage; and
at least one securement feature formed on the tray and configured to secure at least a portion of an intravenous catheter or a syringe.

17. The intravenous catheter securement device of claim 16, wherein at least one securement feature is at least one slot.

18. The intravenous catheter securement device of claim 16, further comprising at least one tearable or easily removable strap removably secured to the holder.

19. The intravenous catheter securement device of claim 18, wherein the at least one tearable or easily removable strap has at least one opening configured to receive at least a portion of bodily appendage.

20. The intravenous catheter securement device of claim 16, wherein the tray has a foam insert in at least a portion of the interior cavity.
